# EUROPEAN PATENT APPLICATION

(11) **EP 4 643 781 A1**
(43) Date of publication of application: **05.11.2025**
(21) Application number: 23912084.3
(22) Date of filing: 25.12.2023
(51) Int. Cl.: A61B 6/00, A61B 6/46

(54) **INFORMATION PROCESSING DEVICE, RADIATION IRRADIATION DEVICE, INFORMATION PROCESSING METHOD, AND INFORMATION PROCESSING PROGRAM**

(30) Priority: 27.12.2022 JP 2022210691
(71) Applicant: FUJIFILM Corporation, Tokyo 106-8620 (JP)
(72) Inventor: SUGAHARA, Masataka, Tokyo 106-8620 (JP); KOBAYASHI, Takeyasu, Tokyo 106-8620 (JP); ONODA, Atsushi, Tokyo 106-8620 (JP)
(74) Representative: Dehns Germany Partnerschaft mbB
(86) International application number: PCT/JP2023/046516
(87) International publication number: WO 2024/143305

(57) **Abstract**

An information processing device includes at least one processor, in which the processor is configured to: acquire an optical image obtained by optically imaging a subject; specify a position of a region of interest based on the optical image; and perform control of displaying a first marker indicating a center position of an irradiation field of radiation and a second marker indicating a position determined in advance for the specified region of interest, in a case in which the subject is radiographically imaged from a direction that is substantially the same as an imaging direction of the optical imaging, on a display in a form of being superimposed on the optical image.

## Description

### Technical Field

The present disclosure relates to an information processing device, a radiation irradiation apparatus, an information processing method, and an information processing program.

### Background Art

In the related art, in radiography, a technology of supporting positioning based on an optical image obtained by optically imaging a subject is known. For example, JP2014-117368A discloses a technology of guiding reproduction of imaging under the same imaging conditions and the same positioning as in past imaging at a present point in time based on an optical image of a subject and imaging conditions at a past point in time.

### SUMMARY OF INVENTION

In recent years, there has been a demand for a technology of supporting imaging in accordance with positioning determined in advance in guidelines or the like in order to stabilize a quality of a radiation image.

The present disclosure provides an information processing device, a radiation irradiation apparatus, an information processing method, and an information processing program that can support positioning in radiography.

A first aspect of the present disclosure relates to an information processing device comprising: at least one processor, in which the processor is configured to: acquire an optical image obtained by optically imaging a subject; specify a position of a region of interest based on the optical image; and perform control of displaying a first marker indicating a center position of an irradiation field of radiation and a second marker indicating a position determined in advance for the specified region of interest, in a case in which the subject is radiographically imaged from a direction that is substantially the same as an imaging direction of the optical imaging, on a display in a form of being superimposed on the optical image.

In the first aspect, the processor may be configured to: acquire an imaging order determined in advance for a type of a region of interest of an imaging target; and perform control of displaying the second marker indicating a position determined in advance in accordance with the type of the region of interest determined in the imaging order, on the display in a form of being superimposed on the optical image.

In the first aspect, the processor may be configured to: receive an input of a type of a region of interest of an imaging target; and perform control of displaying the second marker indicating a position determined in advance in accordance with the input type of the region of interest, on the display in a form of being superimposed on the optical image.

In the first aspect, the processor may be configured to: perform control of displaying a third marker indicating a detection region of the radiation in a radiation detector that detects the radiation, which has been transmitted through the subject, to generate a radiation image of the subject, on the display in a form of being superimposed on the optical image.

In the first aspect, the processor may be configured to: acquire distance information indicating a distance between a radiation source of the radiation and the radiation detector; derive a size of the detection region based on the distance information; and perform control of displaying the third marker corresponding to the derived size of the detection region, on the display in a form of being superimposed on the optical image.

In the first aspect, the processor may be configured to: specify a position of the radiation detector in the optical image; and perform control of displaying the third marker corresponding to the specified position of the radiation detector, on the display in a form of being superimposed on the optical image.

In the first aspect, the processor may be configured to: acquire the optical image obtained by optically imaging the radiation detector to which a marker indicating the detection region of the radiation is added, together with the subject; and specify the position of the radiation detector in the optical image based on the marker included in the optical image.

In the first aspect, the processor may be configured to: specify the position of the radiation detector in the optical image using a positioning sensor provided in the radiation detector.

In the first aspect, the processor may be configured to: acquire a representative optical image obtained by optically imaging the subject within a predetermined period including a point in time at which the subject is irradiated with the radiation; acquire a radiation image of the subject from a radiation detector that detects the radiation, which has been transmitted through the subject, to generate the radiation image; perform control of displaying the representative optical image on the display in a first period from the acquisition of the representative optical image to the acquisition of the radiation image; and perform control of displaying the radiation image on the display in a second period after the acquisition of the radiation image.

In the first aspect, the processor may be configured to: perform control of displaying a region in the representative optical image corresponding to a region included in the radiation image, on the display in the first period.

In the first aspect, the processor may be configured to: acquire information indicating an irradiation period from a start point in time to an end point in time of the irradiation of the subject with the radiation; instruct an imaging apparatus of the representative optical image to perform the optical imaging of the subject at a shutter speed corresponding to the irradiation period; and acquire the representative optical image obtained by optically imaging the subject at the shutter speed corresponding to the irradiation period from the imaging apparatus.

In the first aspect, the processor may be configured to: store the acquired optical image in a storage unit; and delete the optical image stored in the storage unit after a radiation image of the subject is acquired from a radiation detector that detects the radiation, which has been transmitted through the subject, to generate the radiation image.

A second aspect of the present disclosure relates to a radiation irradiation apparatus comprising: the information processing device according to the first aspect; a radiation source that irradiates the subject with radiation; and an imaging apparatus that optically images the subject, in which the radiation irradiation apparatus is portable.

A third aspect of the present disclosure relates to an information processing method comprising: acquiring an optical image obtained by optically imaging a subject; specifying a position of a region of interest based on the optical image; and performing control of displaying a first marker indicating a center position of an irradiation field of radiation and a second marker indicating a position determined in advance for the specified region of interest, in a case in which the subject is radiographically imaged from a direction that is substantially the same as an imaging direction of the optical imaging, on a display in a form of being superimposed on the optical image.

A fourth aspect of the present disclosure relates to an information processing program causing a computer to execute a process comprising: acquiring an optical image obtained by optically imaging a subject; specifying a position of a region of interest based on the optical image; and performing control of displaying a first marker indicating a center position of an irradiation field of radiation and a second marker indicating a position determined in advance for the specified region of interest, in a case in which the subject is radiographically imaged from a direction that is substantially the same as an imaging direction of the optical imaging, on a display in a form of being superimposed on the optical image.

According to the above-described aspects, the information processing device, the radiation irradiation apparatus, the information processing method, and the information processing program of the present disclosure can support the positioning in the radiography.

### BRIEF DESCRIPTION OF DRAWINGS

Fig. 1 is a diagram showing an example of a schematic configuration of an imaging system.
Fig. 2 is a perspective view showing an example of a usage aspect of a radiation irradiation apparatus.
Fig. 3 is an external perspective view showing an example of a configuration of the radiation irradiation apparatus.
Fig. 4 is an external perspective view showing an example of the configuration of the radiation irradiation apparatus.
Fig. 5 is a block diagram showing an example of a hardware configuration of the radiation irradiation apparatus.
Fig. 6 is a diagram showing processing contents of a control device.
Fig. 7 is an example of a screen displayed on a display.
Fig. 8 is an example of the screen displayed on the display.
Fig. 9 is an example of the screen displayed on the display.
Fig. 10 is an example of the screen displayed on the display.
Fig. 11 is an example of another form of a first marker and a second marker.
Fig. 12 is an example of the screen displayed on the display.
Fig. 13 is an example of the screen displayed on the display.
Fig. 14 is a flowchart showing an example of control processing.

### DESCRIPTION OF EMBODIMENTS

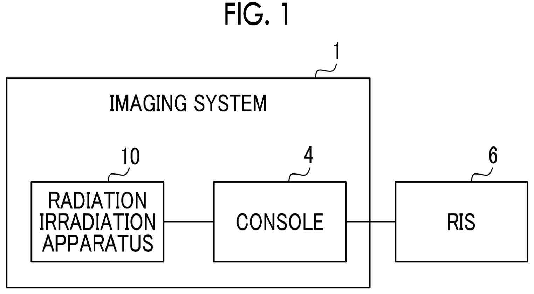

Hereinafter, an embodiment of the present disclosure will be described in detail with reference to the accompanying drawings. First, a configuration of an imaging system 1 will be described with reference to Fig. 1. Fig. 1 is a diagram showing an example of the schematic configuration of the imaging system 1. As shown in Fig. 1, the imaging system 1 comprises a radiation irradiation apparatus 10 and a console 4. The radiation irradiation apparatus 10 and the console 4, and the console 4 and an external radiology information system (RIS) 6 are configured to be connected to each other via a wired or wireless network.

The console 4 acquires an imaging order and the like from the RIS 6, and controls the radiation irradiation apparatus 10 in accordance with the acquired imaging order, an instruction of a user, and the like. The radiation irradiation apparatus 10 captures a radiation image of a subject in accordance with the control of the console 4, the instruction of the user, and the like.

Next, the radiation irradiation apparatus 10 will be described. It should be noted that, in the following description, for convenience of description, a width direction, a front-rear direction (also referred to as a depth direction), and a height direction of the radiation irradiation apparatus 10 are indicated by three arrows X, Y, and Z, respectively. First, the height direction is indicated by the arrow Z, a direction indicated by the arrow Z is an up direction of the radiation irradiation apparatus 10, and an opposite direction of the up direction is a down direction. The height direction is a vertical direction. The width direction is indicated by the arrow X perpendicular to the arrow Z, a direction indicated by the arrow X is a right direction of the radiation irradiation apparatus 10, and an opposite direction of the right direction is a left direction. The front-rear direction is indicated by the arrow Y perpendicular to the arrow Z and the arrow X, a direction indicated by the arrow Y is a front direction of the radiation irradiation apparatus 10, and an opposite direction of the front direction is a rear direction. That is, in the radiation irradiation apparatus 10, an irradiation direction of the radiation is the front direction, and a side on which a subject A stands (see Fig. 1) is the front direction. In addition, hereinafter, the expressions using the side, such as an upper side, a lower side, a left side, a right side, a front side, and a rear side, have the same meaning as the expressions using the direction.

In the present embodiment, a "vertical direction" refers not only to a perfect vertical direction but also to a vertical direction in the sense of including an error that is generally allowed in the technical field to which the technology of the present disclosure belongs and that does not contradict the gist of the technology of the present disclosure. In addition, similarly, "horizontal direction" refers not only to a perfect horizontal direction but also to a horizontal direction in the sense of including an error that is generally allowed in the technical field to which the technology of the present disclosure belongs and that does not contradict the gist of the technology of the present disclosure.

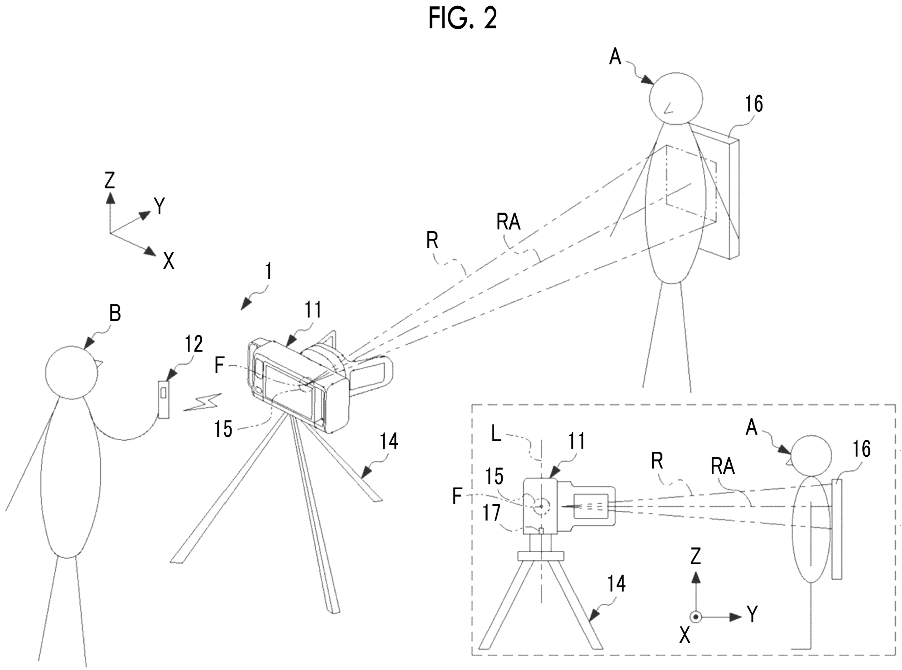

Fig. 2 is a perspective view showing an example of a use aspect of the radiation irradiation apparatus 10. As shown in Fig. 2 as an example, the radiation irradiation apparatus 10 comprises an apparatus body 11 and a remote operation unit 12. The apparatus body 11 is a device that can irradiate the subject A with radiation R. The apparatus body 11 comprises a radiation tube 15, which is a radiation generation source, inside, and irradiates the subject A with radiation (for example, X-rays or γ-rays) generated in the radiation tube 15 through an irradiation field limiter, an irradiation window, and the like. The radiation tube 15 is an example of a "radiation source" according to the technology of the present disclosure. It should be noted that, here, "remote" means separation to the extent caused by physical separation, and does not mean an amount of distance.

The radiation irradiation apparatus 10 has a portable size and weight. That is, the radiation irradiation apparatus 10 is a portable radiation irradiation apparatus. The radiation irradiation apparatus 10 may be used, for example, in a simple radiographic examination at a medical facility, or may be used in a radiographic examination during home medical care. In addition, the radiation irradiation apparatus 10 may be used outdoors. For example, the radiation irradiation apparatus 10 may be used for an on-site medical care in a disaster-stricken area or a medically underserved area.

The apparatus body 11 is set at a position (for example, a height and a distance) determined in advance with respect to the subject A via, for example, a tripod 14. A fixing unit 17 for fixing the tripod 14 and the apparatus body 11 is provided on a lower surface of the apparatus body 11. The fixing unit 17 is, for example, a screw hole. The fixing unit 17 is located on a straight line L that is perpendicular to a central axis RA of a flux of the radiation R and that passes through a focal point F of the radiation tube 15. The radiation tube 15 generates the radiation R, for example, by causing electrons emitted from a cathode to collide with a target. The focal point F is a position at which the electrons collide with the target. The flux of the radiation R spreads in a conical shape with the focal point F as a base point. The central axis RA is a central axis of such a flux. The fixing unit 17 is provided at a position where the straight line L and the lower surface of the apparatus body 11 intersect. In the radiation irradiation apparatus 10, a portion in which the focal point F of the radiation tube 15 is located is close to a centroid. By providing the fixing unit 17 on the straight line L, it is easy to stabilize the radiation irradiation apparatus 10 on the tripod 14.

The remote operation unit 12 is a device that can remotely operate the apparatus body 11. The remote operation unit 12 is attachable to and detachable from the apparatus body 11. The remote operation unit 12 remotely operates the apparatus body 11, for example, by performing wireless communication with the apparatus body 11. The remote operation by the remote operation unit 12 includes, for example, an operation of causing the apparatus body 11 to irradiates the subject A with the radiation R.

A user B, who is an operator of the radiation irradiation apparatus 10, takes out the remote operation unit 12 from the apparatus body 11 and then operates the remote operation unit 12 in a state of being separated from the apparatus body 11 by a predetermined distance. As a result, the subject A is irradiated with the radiation R emitted from the radiation tube 15 of the apparatus body 11. The radiation R transmitted through the subject A is detected by a detector 16. The detector 16 is, for example, a so-called flat panel detector, has a detection surface on which pixels are two-dimensionally arranged, and outputs an image signal corresponding to an intensity of the radiation R incident on each pixel. The radiation R is transmitted through the subject A to carry information on an internal tissue of the subject A. The detector 16 detects the radiation R in each pixel of the detection surface to output an image signal representing a projection image of the internal tissue of the subject A as a radiation image. The detector 16 is an example of a "radiation detector" according to the technology of the present disclosure.

The user B houses the remote operation unit 12 in the apparatus body 11 after completing imaging using the radiation irradiation apparatus 10. In a state in which the remote operation unit 12 is housed in the apparatus body 11, the radiation irradiation apparatus 10 is carried by the user B or is stored in a storage case of the radiation irradiation apparatus 10.

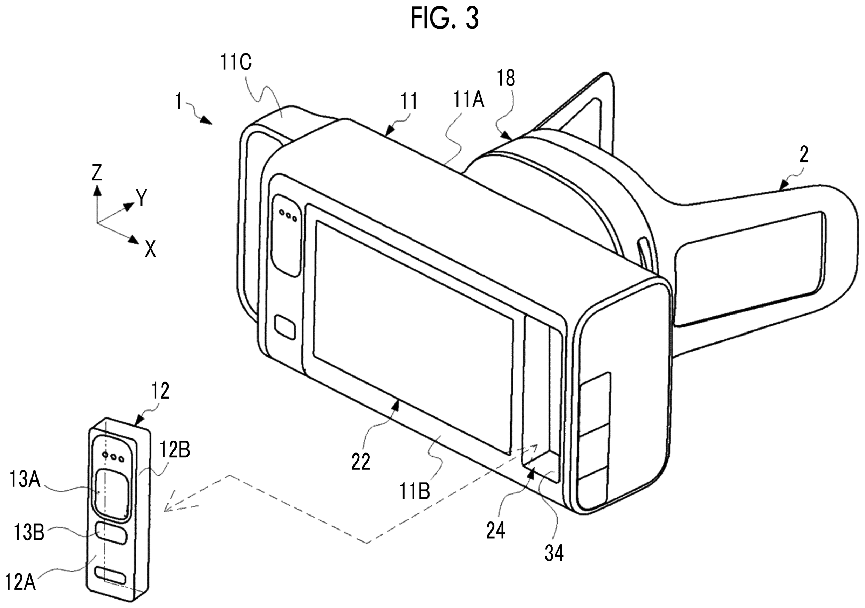

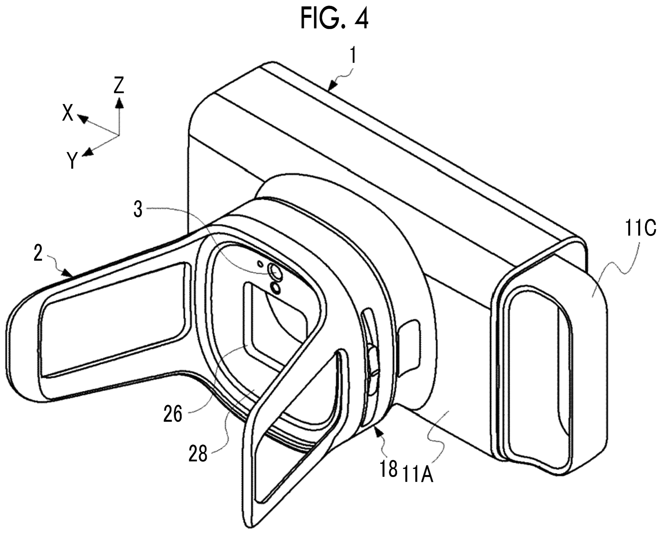

Figs. 3 and 4 are external perspective views showing an example of a configuration of the radiation irradiation apparatus 10. As shown in Fig. 3 as an example, the apparatus body 11 has a substantially rectangular parallelepiped shape having a longitudinal direction in a left-right direction. A tubular portion 18 that protrudes toward the irradiation direction of the radiation R is provided on a front surface 11A of the apparatus body 11. The irradiation field limiter and the irradiation window, which will be described below, are attached to the inside of the tubular portion 18. In addition, a skin guard 20 is attached to a distal end of the tubular portion 18. The skin guard 20 is used to ensure a necessary space between the apparatus body 11 and the subject A, and prevents the subject A from being irradiated with the radiation R in a state in which the apparatus body 11 is too close to the subject A. Further, a holding member 11C is attached to a left side surface of the apparatus body 11. The user B holds the radiation irradiation apparatus 10 via the holding member 11C.

A housing portion 24 is provided on a rear surface 11B of the apparatus body 11. The housing portion 24 can attachably and detachably house the remote operation unit 12 in the rear surface 11B of the apparatus body 11. Specifically, the housing portion 24 has a recessed inner wall surface 34. In a state in which the remote operation unit 12 is housed in the housing portion 24, the inner wall surface 34 faces all surfaces of the remote operation unit 12 except for a back surface 12B. In this way, the housing portion 24 attachably and detachably houses the remote operation unit 12.

A display 22 is provided on the rear surface 11B of the apparatus body 11. The display 22 displays various types of information related to radiography. The display 22 may be, for example, a liquid crystal display or electro-luminescence (EL) display. The display 22 is an example of a "display" according to the technology of the present disclosure.

The remote operation unit 12 has a substantially rectangular parallelepiped shape having a longitudinal direction in an up-down direction in a state of being housed in the apparatus body 11. The remote operation unit 12 has an operation surface 12A and the back surface 12B. The operation surface 12A is provided with an irradiation button 13A and an imaging button 13B. The irradiation button 13A is an operation button for issuing an instruction to perform the irradiation with the radiation R. In a case in which the irradiation button 13A is pressed by the user B, a signal for the irradiation with the radiation R is output from the remote operation unit 12 to the apparatus body 11.

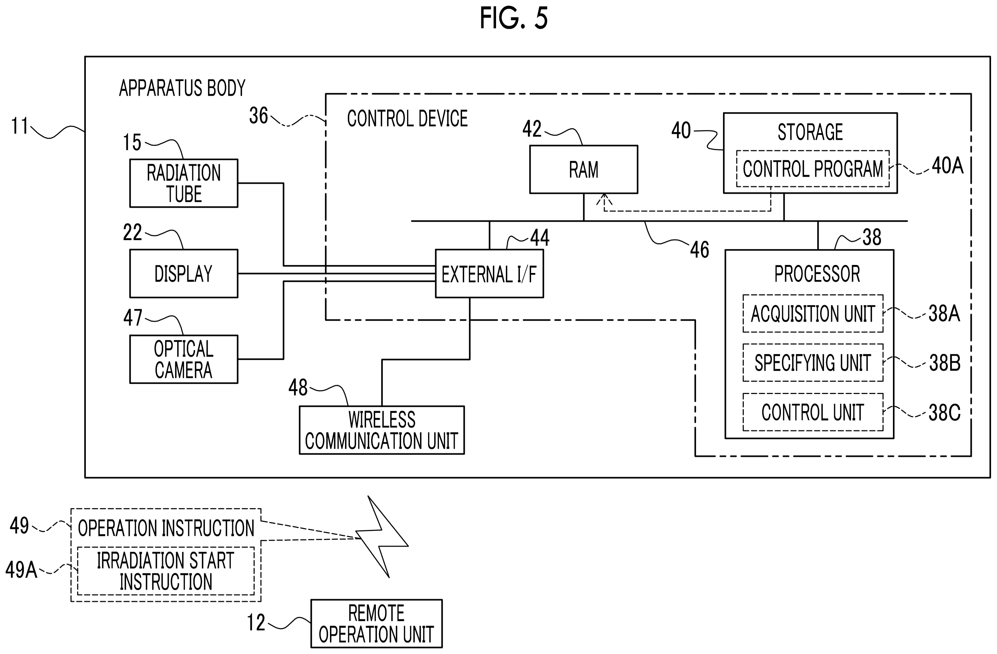

In addition, the radiation irradiation apparatus 10 incorporates an optical camera 47 (see Fig. 5). The imaging button 13B is an operation button for issuing an instruction for imaging using the optical camera 47. In a case in which the imaging button 13B is pressed by the user B, a signal for causing the optical camera 47, which will be described below, to perform imaging is output from the remote operation unit 12 to the apparatus body 11. The back surface 12B is a surface opposite to the operation surface 12A, and operation keys, including the irradiation button 13A and the imaging button 13B, are not provided on the back surface 12B.

It should be noted that, here, the example has been described in which the irradiation button 13A and the imaging button 13B are buttons, but this is merely an example. The irradiation button 13A and the imaging button 13B may be a touch pad in addition to a cursor, a slide switch, and the like.

As shown in Fig. 4 as an example, the tubular portion 18 protruding from the front surface 11A of the apparatus body 11 has the irradiation field limiter 26 and the irradiation window 28. The irradiation field limiter 26 is an irradiation field limiter that defines an irradiation range of the radiation R in a predetermined range, and is also referred to as a collimator. In addition, the irradiation window 28 is a window member that is made of a member transparent to the radiation R, and partitions the outside and an inner side of the tubular portion 18. The radiation R emitted from the radiation tube 15 has the irradiation range defined by the irradiation field limiter 26, and is emitted from the irradiation window toward the subject A.

The optical camera 47 is provided in the tubular portion 18 (see Fig. 5). The optical camera 47 images the subject A, and the optical image obtained by imaging subject A is used for registration of an irradiation position of the radiation R (details will be described below). The optical camera 47 enables still image capturing and moving image capturing. An imaging direction of the optical imaging by the optical camera 47 and an imaging direction of the radiography by the irradiation with the radiation R from the radiation tube 15 are substantially the same direction. Here, the "substantially the same direction" includes a deviation to the extent that the registration with the radiation image can be achieved by subjecting image correction (geometric transformation) such as an affine transformation and a projective transformation to the optical image.

The optical camera 47 is, for example, an imaging apparatus having an image sensor such as a charge coupled device (CCD) image sensor and a complementary metal-oxide-semiconductor (CMOS) image sensor. Reference numeral 30 denotes an imaging window that is a part of a lens of the optical camera 47. Image light of the subject A is incident on the image sensor in the optical camera 47 through the imaging window 30. The optical camera 47 is an example of an "imaging apparatus" according to the technology of the present disclosure.

Fig. 5 is a block diagram showing an example of a hardware configuration of the radiation irradiation apparatus 10. As shown in Fig. 5 as an example, the apparatus body 11 comprises a control device 36. The control device 36 controls an overall operation of the apparatus body 11. The control device 36 comprises a processor 38, a storage 40, a random-access memory (RAM) 42, and an external interface (I/F) 44. The processor 38, the storage 40, the RAM 42, and the external I/F 44 are connected to each other via a bus 46, such as a system bus and a control bus, such that various types of information can be exchanged. The control device 36 is an example of an "information processing device" according to the technology of the present disclosure.

The processor 38 is, for example, a central processing unit (CPU). It should be noted that the processor 38 may be provided with a graphics processing unit (GPU) dedicated to image processing, separately from the CPU. The processor 38 is an example of a "processor" according to the technology of the present disclosure.

The storage 40 is a nonvolatile storage device that stores various programs, various parameters, and the like. Examples of the storage 40 include a flash memory (for example, an electrically erasable and programmable read-only memory (EEPROM) and a solid state drive (SSD)), and/or a hard disk drive (HDD). It should be noted that the flash memory and the HDD are merely an example, and at least one of the flash memory, the HDD, a magnetoresistive memory, or a ferroelectric memory may be used as the storage 40.

A control program 40A in the control device 36 is stored in the storage 40. The control program 40A is an example of an "information processing program" according to the technology of the present disclosure. The storage 40 is an example of a "storage unit" according to the technology of the present disclosure.

The RAM 42 is a memory that stores the information and is used as a work memory by the processor 38. Examples of the RAM 42 include a dynamic random access memory (DRAM) and/or a static random access memory (SRAM).

The processor 38 reads out the control program 40A from the storage 40 to execute the read out control program 40A on the RAM 42.

The external I/F 44 controls the exchange of various types of information with devices present outside the control device 36. The external I/F 44 is connected to the radiation tube 15, the display 22, the optical camera 47, and a wireless communication unit 48 in a communicable manner.

The apparatus body 11 comprises the wireless communication unit 48. The wireless communication unit 48 wirelessly communicates information including an operation instruction 49 with the remote operation unit 12. A wireless communication method is, for example, a communication method based on specifications of Bluetooth (registered trademark). The operation instruction 49 refers to an instruction to remotely operate the apparatus body 11. The operation instruction 49 includes an irradiation start instruction 49A to cause the apparatus body 11 to start the irradiation with the radiation. As another example, the operation instruction 49 includes an instruction to start imaging using the optical camera 47 and/or an instruction to turn off a power of the apparatus body 11. The wireless communication unit 48 is hardware that is used to perform wireless communication with the remote operation unit 12, and is a wireless communication interface (I/F). The wireless communication I/F as the wireless communication unit 48 includes, for example, a communication antenna and a transmission-and-reception circuit.

It should be noted that, here, although Bluetooth (registered trademark) is shown as the wireless communication method between the wireless communication unit 48 and the remote operation unit 12, the technology of the present disclosure is not limited to this. As the first method, Zigbee (registered trademark) or infrared communication may be used.

Hereinafter, an example of a functional configuration of the control device 36 will be described. As shown in Fig. 5, the control device 36 includes an acquisition unit 38A, a specifying unit 38B, and a control unit 38C. In a case in which the processor 38 executes the control program 40A, the processor 38 functions as each of the functional units of the acquisition unit 38A, the specifying unit 38B, and the control unit 38C.

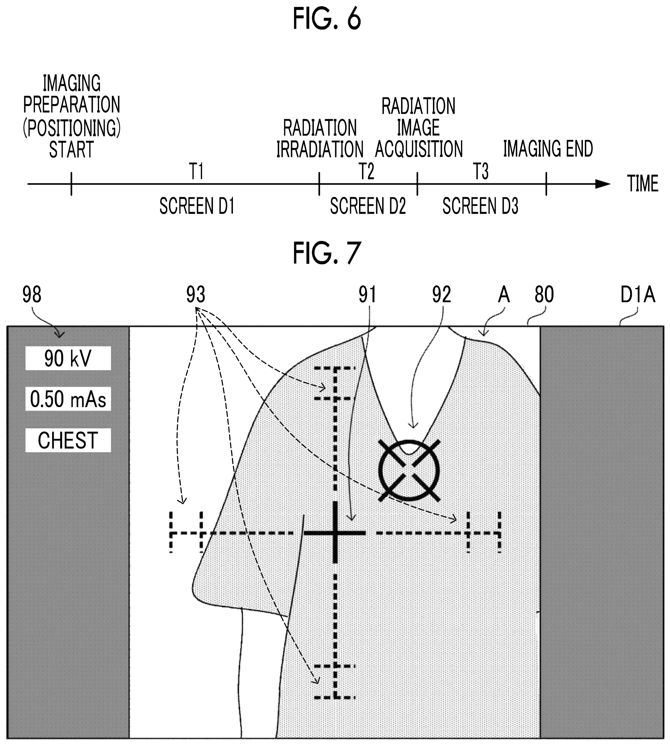

As shown in Fig. 6, the control device 36 according to the present embodiment displays different screens D1 to D3 on the display 22 for each phase of the radiography. Specifically, the control device 36 displays, for example, the screen D1 for supporting positioning on the display 22 in a period T1 from the start of the preparation of the radiography, such as positioning, to the irradiation with the radiation R. In addition, the control device 36 displays the screen D2 for improving the user experience on the display 22 in a period T2 from the irradiation with the radiation R to the acquisition of the radiation image by the detector 16. In addition, the control device 36 displays the screen D3 including the radiation image on the display 22 in a period T3 after the radiation image is acquired from the detector 16. Hereinafter, the functions of the acquisition unit 38A, the specifying unit 38B, and the control unit 38C will be described for each of the periods T1 to T3.

### <Period T1, Screen D1>

First, the functions of the acquisition unit 38A, the specifying unit 38B, and the control unit 38C in the period T1 from the start of the preparation of the radiography, such as the positioning, to the irradiation with the radiation R will be described with reference to Figs. 7 to 10. In the period T1, the control device 36 displays the screen D1 for supporting the positioning on the display 22. Figs. 7 to 10 show screens D1A to D1D as examples of the screen D1 displayed on the display 22 by the control unit 38C.

The acquisition unit 38A acquires an optical image 80 obtained by optically imaging the subject A using the optical camera 47. The specifying unit 38B specifies a position of a region of interest of the subject A based on the optical image 80 acquired by the acquisition unit 38A. The region of interest is a region corresponding to an imaging part. For example, in a case of imaging a lung field, a chest is the region of interest, in a case of imaging a stomach, an abdomen is the region of interest, and in a case of imaging a knee joint, a leg is the region of interest.

Specifically, the acquisition unit 38A may acquire an imaging order determined in advance for a type of the region of interest of the imaging target from the console 4 and/or the RIS 6. In this case, the specifying unit 38B specifies the position of the region of interest determined in the imaging order acquired by the acquisition unit 38A based on the optical image 80.

Further, for example, the acquisition unit 38A may receive an input of the type of the region of interest of the imaging target. For example, an operation unit that allows the user B to input the type of the region of interest may be provided in the remote operation unit 12 and/or the apparatus body 11, and the input of the type of the region of interest of the imaging target may be received by the operation unit. In addition, for example, the console 4 may receive the input of the type of the region of interest of the imaging target, and the acquisition unit 38A may acquire information on the type of the region of interest received by the console 4. In this case, the specifying unit 38B specifies the position of the received region of interest based on the optical image 80.

It should be noted that a known method can be applied as appropriate to the method of specifying the region of interest by the specifying unit 38B. For example, the specifying unit 38B may specify a plurality of joint points of the subject A such as an ear, a shoulder, an elbow, a wrist, a waist, and a knee based on the optical image 80, and specify the position of the region of interest in the optical image 80 based on a relative positional relationship between the plurality of specified joint points. As the method of specifying the joint points, a known posture estimation technology or the like can be applied as appropriate.

As shown in Fig. 7, the control unit 38C performs control of displaying a first marker 91 and a second marker 92 on the display 22 in a form of being superimposed on the optical image 80. The first marker 91 indicates a center position of the irradiation field of the radiation R. The second marker 92 indicates a position determined in advance for the region of interest specified by the specifying unit 38B. The predetermined position for the region of interest is a position at which the positioning is desired in accordance with the center of the irradiation field in the guideline or the like, and is a position predetermined in accordance with the type of the region of interest. For example, in a case in which the region of interest is the chest, the region of interest is the sixth thoracic vertebra (the center of the chest), and in a case in which the region of interest is the entire lower limb, the region of interest is the intermediate position of the left and right knee joints. The position determined in advance for the region of interest is stored in the storage 40 in advance for each type of the region of interest, for example.

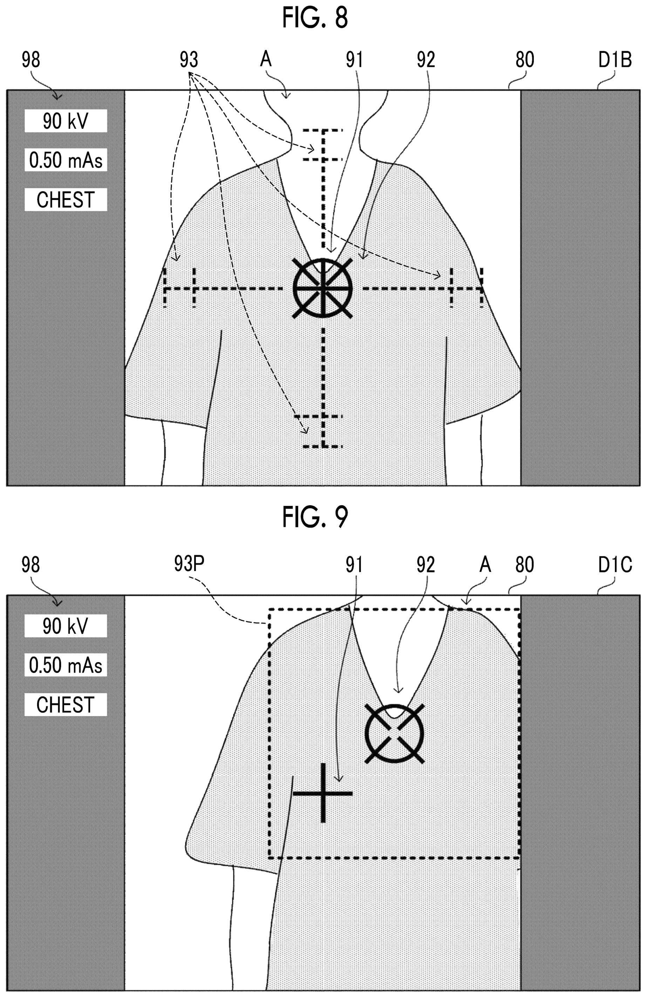

For example, the screen D1A of Fig. 7 shows a state in which the positions of the first marker 91 and the second marker 92 are deviated. In a case in which the radiography is performed in this state, an appropriate radiation image cannot be obtained. In this case, the user B moves the apparatus body 11 of the radiation irradiation apparatus 10 such that the first marker 91 and the second marker 92 overlap each other (see Fig. 8) while checking the display 22. The control unit 38C updates the screen displayed on the display 22 to follow the movement of the apparatus body 11. That is, the acquisition unit 38A acquires the optical image 80 at a predetermined time interval (frame rate), and the specifying unit 38B specifies the position of the region of interest each time the optical image 80 is acquired.

In addition, as shown in Fig. 7, the control unit 38C may perform control of displaying a third marker 93 indicating a detection region of the radiation R in the detector 16 on the display 22 in a form of being superimposed on the optical image 80. Specifically, the control unit 38C may acquire distance information indicating a distance (so-called source to image receptor distance (SID)) between the radiation source of the radiation R (radiation tube 15) and the detector 16, and may derive the size of the detection region based on the distance information. In addition, the control unit 38C may perform control of displaying the third marker 93 corresponding to the derived size of the detection region on the display 22 in a form of being superimposed on the optical image 80.

For example, the size of the detection region may be stored in the storage 40 or the like in advance for each type of the detector 16 that can be used in combination with the radiation irradiation apparatus 10. The control unit 38C may adjust the size of the third marker 93 to be displayed on the display 22 by geometric calculation of the size of the detection region and the distance information stored in the storage 40 or the like. The third marker 93 shown in Fig. 7 indicates the sizes of the detection regions of the two types of detectors 16 that can be used in combination with the radiation irradiation apparatus 10. The third marker 93 allows the user B to determine whether or not the imaging part is appropriately included in the detection region.

It should be noted that, as the distance information, for example, a value of the SID stored in advance in the storage 40 or the like may be used on the premise that the radiation irradiation apparatus 10 is used in a state in which a predetermined appropriate SID is secured. In addition, for example, an actually measured value of the SID measured by a distance measurement sensor such as a laser imaging detection and ranging or light detection and ranging (LIDAR), a time of flight (TOF) camera, and a stereo camera may be used.

It should be noted that the third marker 93 shown in Figs. 7 and 8 is positioned appropriately such that the first marker 91 and the second marker 92 overlap each other as shown in Fig. 8, and thus the third marker 93 matches the position of the actual detection region in the detector 16. That is, as shown in Fig. 7, in a state in which the positions of the first marker 91 and the second marker 92 are deviated and appropriate positioning cannot be performed, the position of the detection region indicated by the third marker 93 does not match the position of the actual detection region in the detector 16.

On the other hand, as shown in Fig. 9, the control unit 38C may perform control of specifying the position of the detector 16 in the optical image 80 and displaying a third marker 93P corresponding to the specified position of the detector 16 on the display 22 in a form of being superimposed on the optical image 80. The third marker 93P shown in Fig. 9 has a position and a size that match the actual detection region.

As the method of specifying the position of the detector 16 in the optical image 80, for example, a method using image recognition may be applied. Specifically, a marker indicating the detection region of the radiation R may be added to the detector 16. In this case, the optical image 80 acquired by the acquisition unit 38A may include the detector 16 to which the marker indicating the detection region of the radiation R is added, together with the subject A. The control unit 38C may specify the position of the detector 16 in the optical image 80 based on the marker indicating the detection region included in the optical image 80.

Meanwhile, in some cases, the marker provided in the detector 16 cannot be detected by image recognition due to the subject A or the like overlapping the detector 16. Therefore, for example, a positioning sensor may be provided in the detector 16, and the control unit 38C may specify the position of the detector 16 in the optical image 80 by using the positioning sensor provided in the detector 16. As the positioning sensor, for example, a known sensor using Bluetooth (registered trademark), a gyro sensor, and the like can be applied.

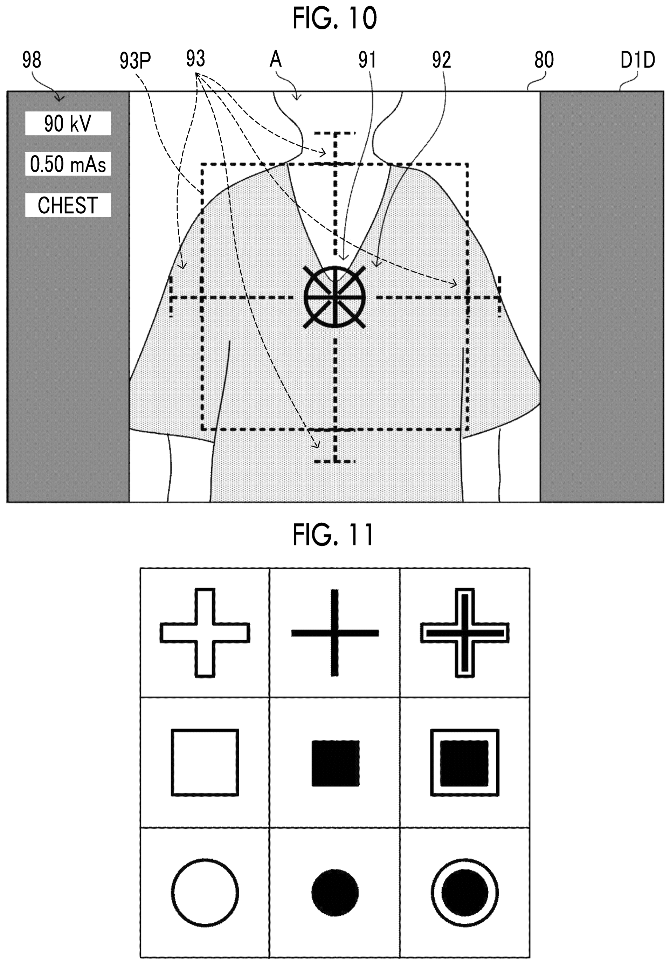

In addition, as shown in Fig. 10, the control unit 38C may perform control of simultaneously displaying the third marker 93 (see Figs. 7 and 8) corresponding to the size of the detection region and the third marker 93P (see Fig. 9) of which the position and the size match the actual detection region, on the display 22. In this case, the positioning can also be supported by the third marker 93 and the third marker 93P.

It should be noted that the forms of the first marker 91, the second marker 92, and the third markers 93 and 93P shown in Figs. 7 to 10 are examples, and the technology of the present disclosure is not limited to this. For example, various types of markers shown in a table of Fig. 11 may be used as the first marker 91 and the second marker 92.

In addition, as shown in Figs. 7 to 10, in a case in which the imaging order is acquired by the acquisition unit 38A, the control unit 38C may perform control of displaying various types of information 98 (imaging condition, imaging part, and the like) included in the imaging order on the display 22.

The user B performs the positioning while checking the first marker 91, the second marker 92, the third marker 93, and the like displayed on the display 22, and issues an instruction to perform the irradiation with the radiation R in a case in which the positioning is completed. In a case in which the user B issues the instruction to perform the irradiation with the radiation R, the control device 36 proceeds to the period T2.

### <Period T2, Screen D2>

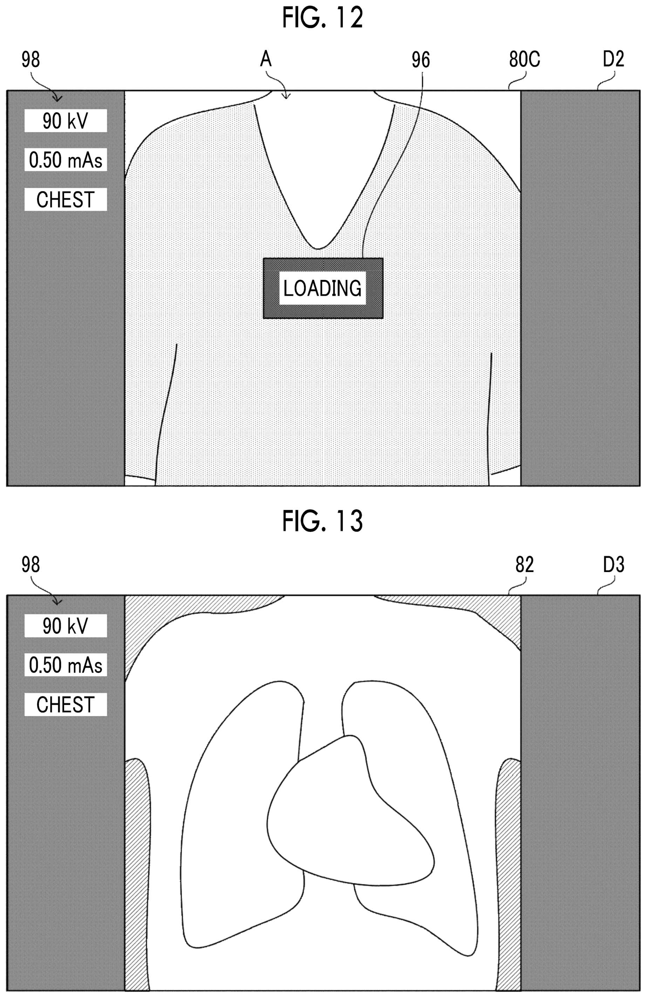

Next, the functions of the acquisition unit 38A and the control unit 38C in the period T2 from the irradiation with the radiation R to the acquisition of the radiation image by the detector 16 will be described with reference to Fig. 12. In the period T2, the control device 36 displays the screen D2 for improving the user experience on the display 22. Specifically, in order for the detector 16 to output the image signal corresponding to the intensity of the radiation R as the radiation image, a waiting time of several seconds to several tens of seconds generally occurs. By displaying the optical image on the display 22 during the waiting time, the reduction of the frustration of the user is achieved, and the user experience is improved. Fig. 12 is an example of the screen D2 displayed on the display 22 by the control unit 38C.

The acquisition unit 38A acquires a representative optical image 80C obtained by optically imaging the subject A using the optical camera 47 within a predetermined period including a point in time at which the subject A is irradiated with the radiation R. The predetermined period including the point in time of the irradiation with the radiation R is, for example, a few seconds before and after the point in time of the irradiation with the radiation R. Preferably, the point in time at which the subject A is irradiated with the radiation R and a point in time at which the subject A is optically imaged are substantially the same.

The control unit 38C performs control of displaying the representative optical image 80C on the display 22 in the period T2 from the irradiation of the subject A with the radiation R to the acquisition of the radiation image by the detector 16. More specifically, the control unit 38C performs control of displaying the representative optical image 80C on the display 22 in a first period from the acquisition of the representative optical image 80C by the acquisition unit 38A to the acquisition of the radiation image by the detector 16. That is, the "first period" according to the technology of the present disclosure corresponds to the period T2 from the irradiation of the subject A with the radiation R to the acquisition of the radiation image by the detector 16.

In this case, it is preferable that the control unit 38C performs control of displaying a region in the representative optical image 80C corresponding to the region included in the radiation image, on the display 22 in the first period. That is, it is preferable that the control unit 38C performs control of trimming the representative optical image 80C captured by the optical camera 47 in accordance with an imaging range of the radiation image and then displaying the representative optical image 80C on the display 22. With this configuration, the user can understand in advance what kind of radiation image can be obtained, and can contribute to improving the user experience.

In addition, as shown in Fig. 12, the control unit 38C may perform control of displaying information 96 indicating that the current point in time is the waiting time (loading) until the radiation image is obtained, on the display 22. It should be noted that the information 96 indicating that the current point in time is the waiting time is not limited to a form of characters as shown in Fig. 12, and may be represented by, for example, symbols and figures.

It should be noted that, in general, the irradiation period from the start point in time to the end point in time of the irradiation of the subject A with the radiation R is several tens of milliseconds to several hundreds of milliseconds, but, in some cases, an appropriate radiation image cannot be obtained due to the subject A moving during the irradiation period. Therefore, the control device 36 may set a shutter speed in the capturing of the representative optical image 80C to match the irradiation time of the radiation R, and thereby being able to use the representative optical image 80C for the examination of the imaging failure factor.

Specifically, the control unit 38C may acquire information indicating the irradiation period from the start point in time to the end point in time of the irradiation of the subject A with the radiation R from the imaging order or the like before the representative optical image 80C is captured (that is, the period T1). In addition, the control unit 38C may instruct the imaging apparatus (optical camera 47) of the representative optical image 80C to perform the optical imaging of the subject A at the shutter speed corresponding to the irradiation period. In addition, the acquisition unit 38A may acquire the representative optical image 80C obtained by optically imaging the subject A using the optical camera 47 at the shutter speed corresponding to the irradiation period.

In a case in which the radiation image is acquired from the detector 16, the control device 36 proceeds to the period T3.

### <Period T3, Screen D3>

Next, the functions of the acquisition unit 38A and the control unit 38C in the period T3 after the acquisition of the radiation image from the detector 16 will be described with reference to Fig. 13. In the period T3, the control device 36 displays the screen D3 including the radiation image on the display 22. Fig. 13 is an example of the screen D3 displayed on the display 22 by the control unit 38C, and includes a radiation image 82.

The acquisition unit 38A acquires the radiation image from the detector 16. The control unit 38C performs control of displaying the radiation image on the display 22 in a second period after the radiation image is acquired by the acquisition unit 38A. The "second period" according to the technology of the present disclosure corresponds to the period T3 after the radiation image is acquired from the detector 16.

The optical image 80 acquired in the period T1 and the representative optical image 80C acquired in the period T2 are stored in the storage 40. From the viewpoint of privacy protection and the like, it may be preferable to delete the optical image 80 and the representative optical image 80C from the storage 40 of the radiation irradiation apparatus 10.

Therefore, the control unit 38C may delete the optical image 80 and/or the representative optical image 80C stored in the storage 40 after the radiation image is acquired from the detector 16. In addition, the control unit 38C may perform control of displaying, on the display 22, a screen for allowing the user to select whether or not to delete the optical image 80 and/or the representative optical image 80C from the storage 40. In this case, the optical image 80 and/or the representative optical image 80C may be deleted from the storage 40 only in a case in which the user issues a delete instruction.

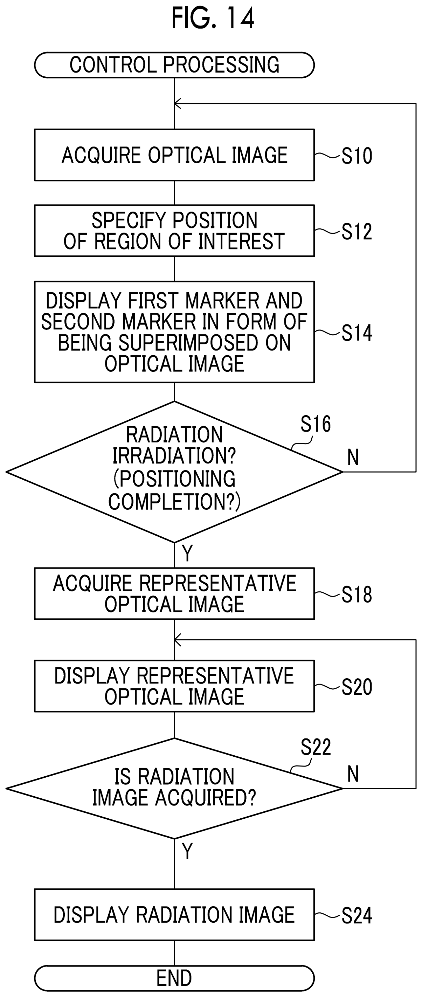

Next, an operation of the control device 36 according to the present embodiment will be described with reference to Fig. 14. In the control device 36, the processor 38 executes the control program 40A to execute control processing shown in Fig. 14. The control processing is executed, for example, in a case in which the user B issues an instruction to start the execution.

In step S10, the acquisition unit 38A acquires the optical image 80 obtained by optically imaging the subject A using the optical camera 47. In step S12, the specifying unit 38B specifies the position of the region of interest of the subject A based on the optical image 80 acquired in step S10. In step S14, the control unit 38C performs control of displaying the first marker 91 indicating the center position of the irradiation field of the radiation R and the second marker 92 indicating the position determined in advance for the region of interest specified in step S12, on the display 22 in a form of being superimposed on the optical image 80.

The acquisition unit 38A, the specifying unit 38B, and the control unit 38C repeat the processing of step S10 to step S14 until the positioning by the user B is completed and the instruction to perform the irradiation with the radiation R is issued (N in step S16). On the other hand, in a case in which the positioning by the user B is completed and the instruction to perform the irradiation with the radiation R is issued, the acquisition unit 38A, the specifying unit 38B, and the control unit 38C proceeds to step S18 (step S16 is Y).

In step S18, the acquisition unit 38A acquires the representative optical image 80C obtained by optically imaging the subject A using the optical camera 47 within the predetermined period including the point in time at which the subject A is irradiated with the radiation R. In step S20, the control unit 38C performs control of displaying the representative optical image 80C acquired in step S18 on the display 22. More specifically, the control unit 38C performs control of continuing displaying the representative optical image 80C on the display 22 in a period until the acquisition unit 38A acquires the radiation image from the detector 16 (N in Step S22).

On the other hand, in a case in which the acquisition unit 38A acquires the radiation image from the detector 16 in step S22, the processing proceeds to step S24. In step S24, the control unit 38C performs control of displaying the radiation image acquired in step S22 on the display 22, and ends the control processing.

As described above, the control device 36 according to the aspect of the present disclosure comprises: at least one processor, in which the processor is configured to: acquire an optical image obtained by optically imaging a subject; specify a position of a region of interest based on the optical image; and perform control of displaying a first marker indicating a center position of an irradiation field of radiation and a second marker indicating a position determined in advance for the specified region of interest, in a case in which the subject is radiographically imaged from a direction that is substantially the same as an imaging direction of the optical imaging, on a display in a form of being superimposed on the optical image. That is, with the control device 36 according to the present embodiment, the positioning in the radiography can be supported by using the first marker and the second marker such that the imaging can be performed in accordance with the positioning determined in advance by the guideline or the like.

In addition, in the control device 36 according to the aspect of the present disclosure, the processor is further configured to: acquire a representative optical image obtained by optically imaging the subject within a predetermined period including a point in time at which the subject is irradiated with the radiation; acquire a radiation image of the subject from a radiation detector that detects the radiation, which has been transmitted through the subject, to generate the radiation image; perform control of displaying the representative optical image on the display in a first period from the acquisition of the representative optical image to the acquisition of the radiation image; and perform control of displaying the radiation image on the display in a second period after the acquisition of the radiation image. That is, by displaying the representative optical image on the display during the waiting time until the radiation detector outputs the radiation image, it is possible to achieve the reduction of the frustration of the user and to improve the user experience.

It should be noted that, in the above-described embodiment, the form has been described in which the control device 36 included in the radiation irradiation apparatus 10 is an example of an information processing device according to the present disclosure, but the present disclosure is not limited to this. For example, an external apparatus such as the console 4 may function as the information processing device according to the present disclosure having the functions of the acquisition unit 38A, the specifying unit 38B, and the control unit 38C.

In the above embodiment, the form example has been described in which the remote operation unit 12 and the apparatus body 11 perform the wireless communication, but the technology of the present disclosure is not limited to this. The remote operation unit 12 and the apparatus body 11 may perform the wired communication.

In addition, in each of the above-described embodiments, for example, as hardware structures of processing units that execute various types of processing, such as the acquisition unit 38A, the specifying unit 38B, the control unit 38C, various processors shown below can be used. As described above, in addition to the CPU that is a general-purpose processor that executes software (program) to function as various processing units, the various processors include a programmable logic device (PLD) that is a processor whose circuit configuration can be changed after manufacture, such as a field programmable gate array (FPGA), and a dedicated electric circuit that is a processor whose circuit configuration is designed for exclusive use in order to execute specific processing, such as an application-specific integrated circuit (ASIC).

One processing unit may be configured by one of the various processors or may be configured by a combination of two or more processors of the same type or different types (for example, a combination of a plurality of FPGAs or a combination of a CPU and an FPGA). Moreover, a plurality of processing units may be configured by one processor.

A first example of the configuration in which the plurality of processing units are configured by one processor is a form in which one processor is configured by a combination of one or more CPUs and the software and this processor functions as the plurality of processing units, as represented by computers, such as a client and a server. Second, as represented by a system-on-chip (SoC) or the like, there is a form in which the processor is used in which the functions of the entire system which includes the plurality of processing units are implemented by a single integrated circuit (IC) chip. In this way, as the hardware structure, the various processing units are configured by one or more of the various processors described above.

Further, as the hardware structure of these various processors, more specifically, it is possible to use an electric circuit (circuitry) in which circuit elements such as semiconductor elements are combined.

In addition, in the above-described embodiment, the aspect has been described in which the control program 40A in the control device 36 is stored in the storage 40 in advance, but the present invention is not limited to this. The control program 40A may be provided in a form of being recorded on a recording medium, such as a compact disc read-only memory (CD-ROM), a digital versatile disc read-only memory (DVD-ROM), and a universal serial bus (USB) memory. In addition, the control program 40A may be provided in a form of being downloaded from the external apparatus through a network. Furthermore, the technology of the present disclosure includes, in addition to the program, a storage medium that stores the program in a non-transitory manner.

In the technology of the present disclosure, the embodiment and the examples described above can be combined as appropriate. The above-described contents and the above-shown contents are detailed descriptions for parts according to the technology of the present disclosure, and are merely examples of the technology of the present disclosure. For example, the above description related to the configuration, the function, the operation, and the effect is the description related to the examples of the configuration, the function, the operation, and the effect of the parts according to the technology of the present disclosure. As a result, it goes without saying that unnecessary parts may be deleted, new elements may be added, or replacements may be made with respect to the above-described contents and the above-shown contents within a range that does not deviate from the gist of the technology of the present disclosure.

In regard with the above-described embodiment, the following supplementary notes are further disclosed.

### <Supplementary Note 1>

An information processing device comprising: at least one processor, in which the processor is configured to: acquire an optical image obtained by optically imaging a subject; specify a position of a region of interest based on the optical image; and perform control of displaying a first marker indicating a center position of an irradiation field of radiation and a second marker indicating a position determined in advance for the specified region of interest, in a case in which the subject is radiographically imaged from a direction that is substantially the same as an imaging direction of the optical imaging, on a display in a form of being superimposed on the optical image.

### <Supplementary Note 2>

The information processing device according to supplementary note 1, in which the processor is configured to: acquire an imaging order determined in advance for a type of a region of interest of an imaging target; and perform control of displaying the second marker indicating a position determined in advance in accordance with the type of the region of interest determined in the imaging order, on the display in a form of being superimposed on the optical image.

### <Supplementary Note 3>

The information processing device according to supplementary note 1 or 2, in which the processor is configured to: receive an input of a type of a region of interest of an imaging target; and perform control of displaying the second marker indicating a position determined in advance in accordance with the input type of the region of interest, on the display in a form of being superimposed on the optical image.

### <Supplementary Note 4>

The information processing device according to any one of supplementary notes 1 to 3, in which the processor is configured to: perform control of displaying a third marker indicating a detection region of the radiation in a radiation detector that detects the radiation, which has been transmitted through the subject, to generate a radiation image of the subject, on the display in a form of being superimposed on the optical image.

### <Supplementary Note 5>

The information processing device according to supplementary note 4, in which the processor is configured to: acquire distance information indicating a distance between a radiation source of the radiation and the radiation detector; derive a size of the detection region based on the distance information; and perform control of displaying the third marker corresponding to the derived size of the detection region, on the display in a form of being superimposed on the optical image.

### <Supplementary Note 6>

The information processing device according to supplementary note 4 or 5, in which the processor is configured to: specify a position of the radiation detector in the optical image; and perform control of displaying the third marker corresponding to the specified position of the radiation detector, on the display in a form of being superimposed on the optical image.

### <Supplementary Note 7>

The information processing device according to supplementary note 6, in which the processor is configured to: acquire the optical image obtained by optically imaging the radiation detector to which a marker indicating the detection region of the radiation is added, together with the subject; and specify the position of the radiation detector in the optical image based on the marker included in the optical image.

### <Supplementary Note 8>

The information processing device according to supplementary note 6 or 7, in which the processor is configured to: specify the position of the radiation detector in the optical image using a positioning sensor provided in the radiation detector.

### <Supplementary Note 9>

The information processing device according to any one of supplementary notes 1 to 8, in which the processor is configured to: acquire a representative optical image obtained by optically imaging the subject within a predetermined period including a point in time at which the subject is irradiated with the radiation; acquire a radiation image of the subject from a radiation detector that detects the radiation, which has been transmitted through the subject, to generate the radiation image; perform control of displaying the representative optical image on the display in a first period from the acquisition of the representative optical image to the acquisition of the radiation image; and perform control of displaying the radiation image on the display in a second period after the acquisition of the radiation image.

### <Supplementary Note 10>

The information processing device according to supplementary note 9, in which the processor is configured to: perform control of displaying a region in the representative optical image corresponding to a region included in the radiation image, on the display in the first period.

### <Supplementary Note 11>

The information processing device according to supplementary note 9 or 10, in which the processor is configured to: acquire information indicating an irradiation period from a start point in time to an end point in time of the irradiation of the subject with the radiation; instruct an imaging apparatus of the representative optical image to perform the optical imaging of the subject at a shutter speed corresponding to the irradiation period; and acquire the representative optical image obtained by optically imaging the subject at the shutter speed corresponding to the irradiation period from the imaging apparatus.

### <Supplementary Note 12>

The information processing device according to any one of supplementary notes 1 to 11, in which the processor is configured to: store the acquired optical image in a storage unit; and delete the optical image stored in the storage unit after a radiation image of the subject is acquired from a radiation detector that detects the radiation, which has been transmitted through the subject, to generate the radiation image.

### <Supplementary Note 13>

A radiation irradiation apparatus comprising: the information processing device according to any one of supplementary notes 1 to 12; a radiation source that irradiates the subject with radiation; and an imaging apparatus that optically images the subject, in which the radiation irradiation apparatus is portable.

### <Supplementary Note 14>

An information processing method comprising: acquiring an optical image obtained by optically imaging a subject; specifying a position of a region of interest based on the optical image; and performing control of displaying a first marker indicating a center position of an irradiation field of radiation and a second marker indicating a position determined in advance for the specified region of interest, in a case in which the subject is radiographically imaged from a direction that is substantially the same as an imaging direction of the optical imaging, on a display in a form of being superimposed on the optical image.

### <Supplementary Note 15>

An information processing program causing a computer to execute a process comprising: acquiring an optical image obtained by optically imaging a subject; specifying a position of a region of interest based on the optical image; and performing control of displaying a first marker indicating a center position of an irradiation field of radiation and a second marker indicating a position determined in advance for the specified region of interest, in a case in which the subject is radiographically imaged from a direction that is substantially the same as an imaging direction of the optical imaging, on a display in a form of being superimposed on the optical image.

The disclosure of JP2022-210691 filed on December 27, 2022 is incorporated in the present specification by reference in its entirety. All of the documents, the patent applications, and the technical standards described in the present specification are incorporated into the present specification by reference to the same extent as in a case in which each of the documents, the patent applications, and the technical standards are specifically and individually stated to be described by reference.

## Claims

1. An information processing device comprising at least one processor, wherein the processor is configured to:
acquire an optical image obtained by optically imaging a subject;
specify a position of a region of interest based on the optical image; and
perform control of displaying a first marker indicating a center position of an irradiation field of radiation and a second marker indicating a position determined in advance for the specified region of interest, in a case in which the subject is radiographically imaged from a direction that is substantially the same as an imaging direction of the optical imaging, on a display in a form of being superimposed on the optical image.

2. The information processing device according to claim 1, wherein the processor is configured to:
acquire an imaging order determined in advance for a type of a region of interest of an imaging target; and
perform control of displaying the second marker indicating a position determined in advance in accordance with the type of the region of interest determined in the imaging order, on the display in a form of being superimposed on the optical image.

3. The information processing device according to claim 1, wherein the processor is configured to:
receive an input of a type of a region of interest of an imaging target; and
perform control of displaying the second marker indicating a position determined in advance in accordance with the input type of the region of interest, on the display in a form of being superimposed on the optical image.

4. The information processing device according to claim 1, wherein the processor is configured to perform control of displaying a third marker indicating a detection region of the radiation in a radiation detector that detects the radiation, which has been transmitted through the subject, to generate a radiation image of the subject, on the display in a form of being superimposed on the optical image.

5. The information processing device according to claim 4, wherein the processor is configured to:
acquire distance information indicating a distance between a radiation source of the radiation and the radiation detector;
derive a size of the detection region based on the distance information; and
perform control of displaying the third marker corresponding to the derived size of the detection region, on the display in a form of being superimposed on the optical image.

6. The information processing device according to claim 4, wherein the processor is configured to:
specify a position of the radiation detector in the optical image; and
perform control of displaying the third marker corresponding to the specified position of the radiation detector, on the display in a form of being superimposed on the optical image.

7. The information processing device according to claim 6, wherein the processor is configured to:
acquire the optical image obtained by optically imaging the radiation detector to which a marker indicating the detection region of the radiation is added, together with the subject; and
specify the position of the radiation detector in the optical image based on the marker included in the optical image.

8. The information processing device according to claim 6, wherein the processor is configured to specify the position of the radiation detector in the optical image using a positioning sensor provided in the radiation detector.

9. The information processing device according to claim 1, wherein the processor is configured to:
acquire a representative optical image obtained by optically imaging the subject within a predetermined period including a point in time at which the subject is irradiated with the radiation;
acquire a radiation image of the subject from a radiation detector that detects the radiation, which has been transmitted through the subject, to generate the radiation image;
perform control of displaying the representative optical image on the display in a first period from the acquisition of the representative optical image to the acquisition of the radiation image; and
perform control of displaying the radiation image on the display in a second period after the acquisition of the radiation image.

10. The information processing device according to claim 9, wherein the processor is configured to perform control of displaying a region in the representative optical image corresponding to a region included in the radiation image, on the display in the first period.

11. The information processing device according to claim 9, wherein the processor is configured to:
acquire information indicating an irradiation period from a start point in time to an end point in time of the irradiation of the subject with the radiation;
instruct an imaging apparatus of the representative optical image to perform the optical imaging of the subject at a shutter speed corresponding to the irradiation period; and
acquire the representative optical image obtained by optically imaging the subject at the shutter speed corresponding to the irradiation period from the imaging apparatus.

12. The information processing device according to claim 1, wherein the processor is configured to:
store the acquired optical image in a storage unit; and
delete the optical image stored in the storage unit after a radiation image of the subject is acquired from a radiation detector that detects the radiation, which has been transmitted through the subject, to generate the radiation image.

13. A radiation irradiation apparatus comprising:
the information processing device according to any one of claims 1 to 12;
a radiation source that irradiates the subject with radiation; and
an imaging apparatus that optically images the subject,
wherein the radiation irradiation apparatus is portable.

14. An information processing method comprising:
acquiring an optical image obtained by optically imaging a subject;
specifying a position of a region of interest based on the optical image; and
performing control of displaying a first marker indicating a center position of an irradiation field of radiation and a second marker indicating a position determined in advance for the specified region of interest, in a case in which the subject is radiographically imaged from a direction that is substantially the same as an imaging direction of the optical imaging, on a display in a form of being superimposed on the optical image.

15. An information processing program causing a computer to execute a process comprising:
acquiring an optical image obtained by optically imaging a subject;
specifying a position of a region of interest based on the optical image; and
performing control of displaying a first marker indicating a center position of an irradiation field of radiation and a second marker indicating a position determined in advance for the specified region of interest, in a case in which the subject is radiographically imaged from a direction that is substantially the same as an imaging direction of the optical imaging, on a display in a form of being superimposed on the optical image.
